# EUROPEAN PATENT SPECIFICATION

(11) **EP 1 569 628 B1**
(45) Date of publication and mention of the grant of the patent: **22.07.2009**
(21) Application number: 03785727.3
(22) Date of filing: 04.12.2003
(51) Int. Cl.: A61K 31/01, A61K 31/07, A61K 31/353, A61K 31/375, A61P 5/00

(54) **NOVEL USE OF LYCOPENE FOR THE TREATMENT OF DISEASES ASSOCIATED WITH ANDROGEN SIGNALLING**
NEUE VERWENDUNG VON LYCOPIN ZUR BEHANDLUNG VON ERKRANKUNGEN IM ZUSAMMENHANG MIT ANDROGENWIRKUNG
NOUVELLE UTILISATION DU LYCOPENE POUR LE TRAITEMENT DE MALADIES ASSOCIÉES AUX ANDROGÈNES

(30) Priority: 06.12.2002 EP 02027244
(43) Date of publication of application: 07.09.2005
(73) Proprietor: DSM IP Assets B.V., 6411 TE Heerlen (NL)
(72) Inventor: BARELLA, Luca, CH-4054 Basel (CH); GORALCZYK, Regina, 79639 Grenzach-Wyhlen (DE); SILER, Ulrich, 79639 Grenzach-Wyhlen (DE); WERTZ, Karin, 79618 Rheinfelden (DE)
(74) Representative: Grossner, Lutz
(86) International application number: PCT/EP2003/013665
(87) International publication number: WO 2004/052351

(56) References cited:
- EP-A- 1 314 438
- WO-A-01/22958
- WO-A-02/058683
- DE-A- 10 109 798
- US-A1- 2002 001 632
- US-A1- 2002 155 163
- PARAN ESTHER ET AL: "Effect of tomato's lycopene on blood pressure, serum lipoproteins, plasma homocysteine and oxidative stress markers in grade I hypertensive patients" AMERICAN JOURNAL OF HYPERTENSION, vol. 14, no. 4 Part 2, April 2001 (2001-04), page 141A XP002276573 Sixteenth Annual Scientific Meeting of the American Society of Hypertension;San Francisco, California, USA; May 15-19, 2001 ISSN: 0895-7061

## Description

The present invention relates to a novel use of lycopene. More particularly, the invention relates to the use of lycopene for prevention, incidence risk reduction, coadjuvant treatment or treatment of non-cancerous symptoms and/or pathologies, which are associated with, favored by or caused by androgen signalling, or which are sensitive to a reduction of androgen signalling. More specifically, the present invention relates to the use of lycopene in the primary prevention and incidence risk reduction of non-cancerous symptoms and/or pathologies (i.e., the prophylactic supplementation of healthy subjects), in the treatment or the coadjuvant treatment of non-eancerotts symptoms and/or pathologies (i.e. the supplementation as therapy or accompanying a running therapy) and in the secondary prevention of non-cancerous symptoms and/or pathologies (i.e., the supplementation after a successful therapy for the prevention of relapse), which are associated with, favored by or caused by androgen signalling or which are sensitive to a reduction of androgen signalling.

Androgen signalling is elementary for the development and maintenance of a variety of physiological functions. In males as well as in females, androgen signalling within physiological windows is necessary, and signalling intensities outside physiological ranges lead to symptoms or pathologies, whose shape or intensity alter according to androgen signalling level.

In the prostate (within benign prostate tissue), androgen signalling is necessary for the development and maintenance of prostate tissue architecture (Janulis L. et al., Prostate (2000) 43: 195-204, Huynh H. et al., J Endocrinol (2001) 171: 109-18, Kucway R. et all Urol (2002) 167: 2443-7). In humans, the skin is a target of androgen signalling, where hair growth and sebum secretion are under androgen control. Especially female physiology seems to be even more sensitive to altered androgen levels. An increased synthesis of androgen in the ovaries (Stein-Leventhal syndrome associated with polycystic ovary syndrome or amenorrhea) or in adrenals (due to carcinogenesis or hyperplasia), or a sensitization due to increased local metabolization within the skin, shows up clinically in dermatological symptoms like male-like hair growth (hirsutism) (Kelestimur F., J Pediatr Endocrinol Metab (2001) 14 Suppl 5:1309-15; discussion 1317), alopecia (Mulinari-Brenner F & Bergfeld WF, Dermatol Nurs (2001) 13: 269-72, 277-8) and feminine acne (Vexiau P. et al., Ann Dermatol Venereol (2002) 129: 174-8, and Gynecol Obstet Fertil (2002) 30: 11-21). Polycystic ovary syndrome (PCOS) is the most frequent androgen disorder of ovarian function. PCOS women are predisposed to infertility due to the chronic anovulation. At the same time they have been found to be profoundly insulin resistant. This baseline insulin resistance combined with the worsening effect of obesity (which may affect up to 75% of the US PCOS population), places these women at increased risk for impaired glucose tolerance and most likely diabetes. Women with PCOS tend to have elevated triglycerides (perhaps the best lipid marker of insulin resistance), and an unfavorably elevated LDL (low density lipoproteins)/HDL (high density lipoproteins) ratio. A lowered HDL-C levels appear to be the strongest lipid predictor of cardiovascular mortality in women (Legro RS, Mol Cell Endocrinol (2002) 186: 219-25, Pugeat M. et al., Horm Res (2000) 54: 322-6, Jacobs DR Jr. et al, Am J Epidemiol (1990) 131: 32-47 and Wilson PW, et al., Arteriosclerosis (1988) 8: 737-41) indicating an increased risk for cardiovascular diseases in PCOS patients.

WO 01/22958 (Avansis Limited) shows a pharmaceutical composition containing lycopene in combination with a flavinoid, an amino acid, magnesium, ascorbate, and Vitamin E that can be used to prevent or treat vascular disorders.

WO 02/058683 (Lycored Natural Products Industries, Ltd) teaches a composition which lowers blood pressure containing a carotenoid which is selected from the group consisting of lycopene, phytofluene, phytoene, astaxanthin, cathaxanthin, and mixtures thereof.

Paran and Engelhard 2001 Am. J. Hypertension 14(4, part1): 147A teaches that lycopene can lower systolic and diastolic blood pressure.

According to the present invention, it has been found that androgen target gene expression in androgen sensitive target organ tissue, as the endpoint and result of androgen signalling, can be significantly reduced by the administration of lycopene or a combination of lycopene and vitamin E.

The present invention, therefore, in one aspect is concerned with the use of lycopene in the manufacture of a composition for the primary and secondary prevention, incidence risk reduction, coadjuvant treatment or treatment of non-cancerous symptoms and/or pathologies, which are associated with, favored by or caused by androgen signalling, or which are sensitive to a reduction of androgen signalling wherein the symptoms and/or pathologies are selected from the group consisting of: polycystic ovary syndrome, hyperandrogenic chronic anovulation, female fertility, ovarian hyperstimulation syndrome, amenorrhea, obesity, accumulation of abdominal fat, hirsutism, menstrual disorder, hyperandrogenism, congenital adrenal hyperplasia (CAH) and stress induced imbalance of androgen signalling.

In another aspect, the present invention is concerned with a method of prevention or treatment of symptoms or pathologies associated with androgen signalling, which comprises administering to a subject (mammal or non-mammal, human or pet including birds and fish, or mammal or non-mammal farm animal) in need of such treatment for therapy or prophylaxis an effective amount of lycopene.

In still another aspect, the invention is concerned with a method of treating non-cancerous symptoms and/or pathologies sensitive to lycopene comprising administering to a mammal, mammal or non-mammal pets including birds and fish, or mammal or non-mammal farm animal in need of such treatment an amount of lycopene, wherein said amount leads to a reduction of androgen signalling.

In a preferred embodiment of the invention, lycopene is used together with vitamin E and/or vitamin C. Most preferred is a combination of lycopene, vitamin E and vitamin C, The term vitamin E as used herein includes racemic vitamin E (D,L-α-tocopherol) or natural vitamin E, as well as derivatives thereof which have biological vitamin E activity, e.g. carboxylic acid esters, such as vitamin E acetate, propionate, butyrate or succinate;and 6-hdroxy-2,5,7,8-tetramethylchroman-2-carboxylic aid (also called Trolox ®) and Trolox®-lipoate. The term vitamin C as used herein includes derivatives thereof, which have biological vitamin C activity, e.g. esters and salts, such as sodium ascorbate, sodium ascorbyl phosphate, sodium ascorbyl polyphosphates and ascorbyl palmitate.

In a further embodiment of the invention, lycopene or lycopene in combination with vitamin E and/or vitamin C is used together with one or more of the following compounds:
(a) Silymarin (extract from *Silybum marianum*) and/or one or more derivatives thereof (silymarin dihemisuccinate sodium salt) and/or one or more of its four main components (silybin [synonymous with silibinin, and sometimes incorrectly called silybinin] and/or isosilybin and/or silydianin and/or silychristin) and/or one or more derivatives thereof (silybin-dihemisuccinate, disilybin, silybin-phosphatidylcholine complex, silybin-phosphate);
(b) Extract of Saw Palmetto (*Sabal serrulata*, syn. *Serenoa repens*) and/or one or more derivatives thereof and/or one or more of its main components being free fatty acids (lauric acid, oleic acid, myristic acid, palmitic acid and/or one or more derivatives thereof) and/or phytosterols (sitosterol, campesterol, stigmasterol, cycloartenol, sitostanol, campestanol and/or derivatives thereof (long-chain fatty acyl ester, ferrulate ester, glycosides));
(c) Genistein aglycone (4', 5, 7-trihydroxyisoflavone) and/or one or more derivatives thereof (genistein glucosides, genistein sulfates, genistein glucuronides);
(d) Apigenin and/or one or more derivatives thereof;
(e) Quercetin (2-(3,4-dihydroxyphenyl)-3,5,7-trihydroxy-4H-1-benzopyrano-4-one) and/or dihydroquercetin and/or one or more derivatives thereof (quercetine glucosides, quercetin glucuronides, quercetine sulphates, methylquercetin (isohamnetin (3'-O-methylquercetin), tamarixetin(4'-O-methylquercetin));
(f) Myricetin and/or one or more derivatives thereof;
(g) Kampferol and/or one or more derivatives thereof;
(h) Resveratrol (*cis-3,* 4', 5-trihydroxystilbene and/or *trans*-3, 4', 5-trihydroxystilbene) and/or one or more derivatives thereof (resveratrol glucosides, resveratrol sulfates, resveratrol glucuronides);
(i) Curcumin (effects of *Curcuma Longa*) and/or one or more derivatives (demethoxy-curcumin, bis-demethoxycurcumin, sodium curcumionate, bis-demethylcurcumin, tetrahydrocurcumin, hexahydrocurcumin, diacteylcurcumin, triethylcurcumin) thereof and/or one or more of its main components (curcumin (diferuloylmethane), demethoxycurcumin, bisdemethoxycurcumin) and/or derivatives thereof (glucuronides, sulfates),
(j) flufenamic acid and/or one or more derivatives (esters) thereof;
(k) geldanamycin
(l) Extract of *Stephania hernandifolia* and/or one or more derivatives thereof and/or one or more of its components (e.g. 4-demethylhasubanonine, epistephanine) and/or derivatives thereof;
(m) Extract of *Myrica rubra* and/or one or more derivatives thereof and/or one or more of its components beeing diarylheptanoids (Quercetin, myricanone, myricanol, and myricetin) named acerogenin and their glycosides named aceroside and/or derivatives thereof;
(n) Astaxanthin ((3S, 3'S)-3, 3'-dihydxoxy-β, β-carotene-4, 4'-dione) and/or one or more isomers and/or monoesters and/or diesters, preferably esters of saturated alkanoic acids, such as acetic, propionic, palmitic, stearic, and succinic acid, mono-unsaturated fatty acids, such as oleic acid, and poly-unsaturated fatty acids, such as linolic, linoleic, docosahexaenoic, and arachidonic acid;
(o) β-Carotene and/or one or more isomers thereof;
(p) β-Cryptoxanthin ((3R)-β, β-carotene-3-ol) and/or one or more isomers or esters thereof, preferably esters of saturated alkanoic acids, such as acetic, propionic, palmitic, stearic, and succinic acid, mono-unsaturated fatty acids, such as oleic acid, and poly-unsaturated fatty acids, such as linolic, linoleic, docosahexaenoic, and arachidonic acid;
(q) (-)-Epigallocatechin gallate (EGCG) and/or (-)-epicatechin gallate (ECG) and/or one or more derivatives thereof;
(r) Lutein ((3R, 3'R, 6'R)-β, ε, carotene-3, 3'-diol) and/or one or more isomers and/or monoesters and/or diesters, preferably esters of saturated alkanoic acids, such as acetic, propionic, palmitic, stearic, and succinic acid, mono-unsaturated fatty acids, such as oleic acid, and poly-unsaturated fatty acids, such as linolic, linoleic, docosahexaenoic, and arachidonic acid, thereof;
(s) Rhizoxin and/or one or more derivatives thereof (palmitoyl rhizoxin);
(t) Vitamin A and/or retinoic acids (*all-trans* retinoic acid and/or *13-cis* retinoic acid and/or *9-cis* retinoic acid) and/or one or more derivatives thereof (*all-trans* retinoic acid, *13-cis* retinoic acid, all-*trans* retinol, *9-cis* retinoic acid or 4-hydroxyphenylretinamide or retinal esters such as all-*trans* retinyl acetate);
(u) Vitamin D2 or vitamin D3 or I α, 25-dihydroxyvitamin D3 or 25-hydroxyvitamin D3 or 1α, 24R, 25-trihydroxyvitamin D3; or 24, 25-dihydroxyvitamin D3
(v) Zeaxanthin ((3R, 3'R)-β, β-carotene-3, 3'-diol) and/or one or more isomers and stereo-isomers (preferably mesozeaxanthin, 3R,3'S-zeaxanthin) and/or monoesters and/or diesters, preferably esters of saturated alkanoic acids, such as acetic, propionic, palmitic, stearic, and succinic acid, mono-unsaturated fatty acids, such as oleic acid, and poly-unsaturated fatty acids, such as linolic, linoleic, docosahexaenoic, and arachidonic acid, thereof;
(w) Carnosic acid and/or one or more derivatives thereof;
(x) Carnosol and/or one or more derivatives thereof;
(y) Depudecin and/or one or more derivatives thereof;
(z) Eponemycin and/or one or more derivatives thereof;
(aa) Dihydroeponemycin and/or one or more derivatives thereof;
(bb) Epoxomicin and/or one or more derivatives thereof;
(cc) Ergosterol and/or one or more derivatives thereof;
(dd) Fisetin and/or one or more derivatives thereof;
(ee) Fumagillin and/or one or more derivatives thereof;
(ff) Lactacystin and/or one or more derivatives thereof;
(gg) Luteolin and/or one or more derivatives thereof;
(hh) Motuporamine C and/or one or more derivatives thereof;
(ii) Ovalicin and/or one or more derivatives thereof;
(jj) Radicicol and/or one or more derivatives thereof;
(kk) Squalamine and/or one or more derivatives thereof;
(ll) Isoliquiritin, isoliquiritigenin, liquiritigenin and/or one or more derivatives thereof;
(mm) Very-long-chain omega-3 fatty acides (eicosapentaenoic acid [C20; 5, omega-3], decosahexaenoic acid [C22; 6, omega-3], polyunsaturated ω-3 fatty acids);
(nn) Shark cartilage extract.
(oo) Glucosinolate derivatives (Methylsulfinylalkyl glucosinolates, e.g. [1-methylsulfinylmethyl glucosinolate, 2-methylsulfinylethyl glucosinolate, 3-methylsulfinylpropyl glucosinolate (glucoiberin), 4-methylsulfinylbutyl glucosinolate (glucoraphanin), 5-methylsulfinylpentyl glucosinolate (glucoalysin), 6-methylsulfinylhexyl glucosinolate, 7-methylsulfinylheptyl glucosinolate, 8-methylsulfinyloctyl glucosinolate, 9-methylsulfinylnonyl glucosinolate, 10-methylsulfinyldodecyl glucosinolate] or allyl glucosinolate (sinigrin) or phenylethyl glucosinolate (gluconasturtiin) or 3-butenyl glucosinolate (gluconapin) or indol-3-ylmethyl glucosinolate (glucobrassicin) or derivatives thereof [N-methoxyindol-3-ylmethyl glucosinolate (neoglucobrassicin), 4-hydroxyindol-3-ylmethyl glucosinolate (4-OH glucobrassicin), 4-methoxyindol-3-ylmethyl glucosinolate (4-CH₃O glucobrassicin)]).
(pp) Isothiocyanate derivatives (Methylsulfinylalkyl isothiocyanate [1-methylsulfinylmethyl isothiocyanate, 2-methylsulfinylethyl isothiocyanate, 3-methylsulfinylpropyl isothiocyanate, 4-methylsulfinylbutyl isothiocyanate (sulforaphane), 5-methylsulfinylpentyl isothiocyanate, 6-methylsulfinylhexyl isothiocyanate (6-HITC), 7-methylsulfinylheptyl isothiocyanate, 8-methylsulfinyloctyl isothiocyanate, 9-methylsulfinylnonyl isothiocyanate, 10-methylsulfinyldodecyl isothiocyanate] or allyl isothiocyanate or phenylethyl isothiocyanate (PEITC) or 3-butenyl isothiocyanate or indole-3-ylmethylisothiocyanate) or derivatives thereof (N-methoxy indole-3-ylmethylisothiocyanate, 4-hydroxy indole-3-ylmethylisothiocyanate, 4-methoxy indole-3-ylmethylisothiocyanate) or 3-Indolemethanol (Inole-3-carbinol (I3C)).

For the primary and secondary prevention and coadjuvant treatment of non-cancerous symptoms and/or pathologies sensitive to/associated with androgen signalling in accordance with the present invention, lycopene is administered to the subject or a mammal in need of such treatment, i.e. humans, pets or farm animals including birds and fish, in an amount which leads to a reduction of androgen signalling. Such amount is preferably one that results in a plasma concentration of 0.01 to 6 µM (micromolar) and may be within the range of from about 0.0005 mg/kg body weight to about 5 mg/kg body weight per day.

Optionally, lycopene is administered in combination with about 0.2 mg/kg body weight to about 30 mg/kg body weight of vitamin C per day and /or about 0.01 mg/kg body weight to about 15 mg/kg body weight of vitamin E per day.

In accordance with the present invention, lycopene or the combination of lycopene with vitamin C and/or vitamin E may, furthermore, be co-administered together with one or more of following ingredients within dosage ranges set forth below in "mg/kg" or "µg/kg" or "ng/kg" meaning "mg/kg body weight" or "µg/kg body weight" or "ng/kg body weight":

| | | | |
|---|---|---|---|
| Silymarin | 0.01mg/kg | to | 100mg/kg |
| Silybin or equimolar amounts of derivatives | 0.01mg/kg | to | 100mg/kg |
| Isosilybin or equimolar amounts of derivatives | 0.01mg/kg | to | 100mg/kg |
| Silydianin or equimolar amounts of derivatives | 0.01mg/kg | to | 100mg/kg |
| Silychristin or equimolar amounts of derivatives | 0.01mg/kg | to | 100mg/kg |
| Extract of Saw Palmetto (lipophilic (liposterolic) berry extract containing 80 to 90 percent fatty acids) or equimolar amounts of its components mentioned above | 0.01mg/kg | to | 100mg/kg |
| Genistein aglycone | 0.015mg/kg | to | 6mg/kg |
| Apigenin | 0.01mg/kg | to | 500mg/kg |
| Quercetin | 0.001mg/kg | to | 300mg/kg |
| Myricetin | 0.001mg/kg | to | 300mg/kg |
| Kampferol | 0.001mg/kg | to | 300mg/kg |
| Resveratrol or equimolar amounts of derivatives | 0.01mg/kg | to | 1.5mg/kg |
| Curcumin or equimolar amounts of components or derivatives mentioned above | 0.01mg/kg | to | 200mg/kg |
| Flufenamic acid | 0.01mg/kg | to | 200mg/kg |
| Geldanamycin | 0.01mg/kg | to | 200mg/kg |
| Extract of *Stephania hernandifolia*, or equimolar amounts of its components mentioned above | 0.001mg/kg | to | 300mg/kg |
| Extract of *Myrica rubra*, or equimolar amounts of its components mentioned above | 0.001mg/kg | to | 300mg/kg |
| Astaxanthin | 0.001mg/kg | to | 5mg/kg |
| β-Carotene | 0.001mg/kg | to | 5mg/kg |
| β-Cryptoxanthin | 0.001mg/kg | to | 5mg/kg |
| (-)-epigallocatechin gallate (EGCG) or (-)-epicatechin gallate (ECG) | 0.5mg/kg | to | 15mg/kg |
| or equimolar amounts of derivatives mentioned above | | | |
| Lutein | 0.001mg/kg | to | 5mg/kg |
| Rhizoxin | 0.001mg/kg | to | 20mg/kg |
| Palmitoyl Rhizoxin | 0.001mg/kg | to | 20mg/kg |
| Retinoic acid or equimolar amounts of derivatives mentioned above | 0.001mg/kg | to | 5mg/kg |
| All-*trans* Retinol | 3µg/kg | to | 100µg/kg |
| All-*trans* Retinyl acetate | 3.5µg/kg | to | 115µg/kg |
| All-*trans* Retinol palmitate | 5.5µg/kg | to | 180µg/kg |
| Vitamin D2 (Ergocalciferol) | 0.1ng/kg | to | 10µg/kg |
| Vitamin D3 (Cholecalciferol) | 0.1ng/kg | to | 10µg/kg |
| 1α, 25-Dihydroxyvitamin D3 | 0.1ng/kg | to | 0.5µg/kg |
| 25-Hydroxyvitamin D3 | 0.1ng/kg | to | 10µg/kg |
| 1α, 24R, 25-Trihydroxyvitamin D3 | 0.1ng/kg | to | 0.5µg/kg |
| 24, 25-Dihydroxyvitamin D3 | 0.1ng/kg | to | 10µg/kg |
| Zeaxanthin | 0.001mg/kg | to | 5mg/kg |
| Carnosic acid | 0.001mg/kg | to | 250mg/kg |
| Carnosol | 0.001mg/kg | to | 250mg/kg |
| Depudecin | 0.01mg/kg | to | 500mg/kg |
| Eponemycin | 0.01mg/kg | to | 500mg/kg |
| Dihydroeponemycin | 0.01mg/kg | to | 500mg/kg |
| Epoxomicin | 0.01mg/kg | to | 500mg/kg |
| Ergosterol | 0.1mg/kg | to | 2000mg/kg |
| Fisetin | 0.01mg/kg | to | 500mg/kg |
| Fumagillin | 0.1mg/kg | to | 300mg/kg |
| Lactacystin | 0.01mg/kg | to | 250mg/kg |
| Luteolin | 0.01mg/kg | to | 100mg/kg |
| Motuporamine C | 0.1mg/kg | to | 500mg/kg |
| Ovalicin | 0.1mg/kg | to | 250mg/kg |
| Radicicol | 0.1mg/kg | to | 1000mg/kg |
| Squalamine | 0.001 | to | 200mg/kg |
| Isoliquiritin | 1ng/kg | to | 1mg/kg |
| Isoliquiritigenin | 1ng/kg | to | 1mg/kg |
| Very-long-chain omega-3 fatty acides, e.g. eicosapentaenoic acid [C20: 5, omega-3] or equimolar amounts of very-long-chain omega-3 fatty acides mentioned above | 0.001g/kg | to | 0.05g/kg |
| Shark cartilage extract | 0.001g/kg | to | 0.1g/kg |
| Glucosinolate derivatives | 0.01mg/kg | to | 200mg/kg |
| e.g. 4-methylsulfinylbutyl glucosinolate (glucoraphanin) or equimolar amounts of glucosinolate derivatives mentioned above | | | |
| Isothiocyanate derivatives or I3C | 0.001mg/kg | to | 200mg/kg |
| e.g. 4-methylsulfinylbutyl isothiocyanate (sulforaphane) or equimolar amounts of isothiocyanate derivatives mentioned above | | | |

Lycopene or a combination of lycopene, vitamin C and/or vitamin E, optionally together with compounds (a) to (pp) can find use in accordance with the present invention for the completion of human nutrition, nutrition of pets and farm animals, or medical treatment of subjects, especially mammals.

Said compounds may be provided as the active ingredient in compositions, preferably for enteral application, which may be solid or liquid galenical formulations, dietary compositions or animal feed compositions. Examples of solid galenical formulations are tablets, capsules (e.g. hard or soft shell gelatin capsules), pills, sachets, powders, granules and the like which contain the active ingredient together with conventional galenical carriers. Any conventional carrier material can be utilized. The carrier material can be organic or inorganic inert carrier material suitable for oral administration. Suitable carriers include water, gelatin, gum arabic, lactose, starch, magnesium stearate, talc, vegetable oils, and the like. Additionally, additives such as flavouring agents, preservatives, stabilizers, emulsifying agents, buffers and the like may be added in accordance with accepted practices of pharmaceutical compounding. They may also be used in dietary compositions which may be a food, a food premix or a fortified food or a beverage. While the individual active ingredients are suitably administered in a single composition they may also be administered in individual dosage units.

Preferably lycopene is used in accordance with the present invention together with vitamin E, or with vitamin C or with vitamin C and vitamin E. Preferred additional components are as additional active ingredients compound (a), (b), (c), (e), (f), (h), (i), (1), (m), (n), (o), (p), (q), (r), (t), (u), (v), (w), (x), (mm), (oo), (pp), more preferably the active ingredients are (a), (b), (c), (e), (f) (h), (i), (o), (q), (r), (v), (mm) and (pp).

Particularly preferred is the administration of the following active ingredients:
Lycopene, in a concentration so that the daily consumption by a human adult is in the range of from 0.25mg/day to 50mg/day, preferably from 0.2mg/day to 30mg/day; optionally in combination with
Vitamin C or its derivative, in a concentration so that the daily consumption by a human adult is in the range of from 50mg/day to 1000mg/day; and/or
Vitamin E or its derivative, in a concentration so that the daily consumption by a human adult is in the range of from 15mg/day to 600mg/day; and/or
Silymarin (extract from *Silybum marianum*) and/or its four main components (silybin and/or isosilybin and/or silydianin and/or silychristin), in a concentration so that the daily consumption by a human adult of Silymarin or its four main components (silybin, isosilybin, silydianin, silychristin), respectively, is in the range of from 1mg/day to 1000mg/day, preferably from 50mg/day to 800mg/day; and/or
Saw palmetto (lipophilic extract of *Sabal serrulata*, *syn. Serenoa repens*, containing phytosterols and 80 to 90 percent fatty acids) and/or its main components (lauric acid, oleic acid, myristic acid, palmitic acid, sitosterol, campesterol, stigmasterol, cycloartenol, sitostanol, campestanol) and/or derivatives thereof (long-chain fatty acyl ester, ferrulate ester, glycosides)) in a concentration so that the daily consumption by a human adult of saw palmetto or equimolar amounts of its main components is in the range of from 1mg/day to 1000mg/day, preferably from 50mg/day to 250mg/day; and/or
Genistein, in a concentration so that the daily consumption by a human adult is in the range of from 20mg/day to 200mg/day; and/or
Quercetin, in a concentration so that the daily consumption by a human adult is in the range of from 1mg/day to 500mg/day; and/or
Myricetin, in a concentration so that the daily consumption by a human adult is in the range of from 1mg/day to 500mg/day; and/or
Resveratrol, in a concentration so that the daily consumption by a human adult is in the range of from 5 mg/day to 50 mg/day; and/or
Curcumin (effects of *Curcuma Longa*) or equimolar amount of derivatives thereof (demethoxy-curcumin, bis-demethoxycurcumin, sodium curcumionate, bis-demethylcurcumin, tetrahydrocurcumin, hexahydrocurcumin, diacteylcurcumin, triethylcurcumin) and/or equimolar amount of ist main components components (curcumin (diferuloylmethane), demethoxycurcumin, bisdemethoxycurcumin) and/or derivatives thereof (glucuronides, sulfates), in a concentration so that the daily consumption by a human adult is in the range of from 10mg/day to 1000mg/day, preferably from 50mg/day to 800mg/day; and/or
β-Carotene, in a concentration so that the daily consumption by a human adult is in the range of from 0.1 mg/day to 20mg/day, preferably from 2mg/day to 10 mg/day; and/or
(-)-Epigallocatechin gallate (EGCG), in a concentration so that the daily consumption by a human adult is in the range of from 50mg/day to 500mg/day; and/or
Lutein, in a concentration so that the daily consumption by a human adult is in the range of from 0.1mg/day to 50mg/day, preferably from 0.25mg/day to 30mg/day; and/or
Zeaxanthin, in a concentration so that the daily consumption by a human adult is in the range of from 0.1mg/day to 50mg/day, preferably from 0.25mg/day to 30mg/day, and/or
of very-long-chain omega-3 fatty acids, e.g. eicosapentaenoic acid [C20: 5, omega-3] or equimolar amounts of very-long-chain omega-3 fatty acids, in a concentration so that the daily consumption by a human adult is in the range of from 1mg/day to 500mg/day, and/or
Isothiocyanate derivatives or I3C, e.g. 4-methylsulfinylbutyl isothiocyanate (sulforaphane) or equimolar amounts of isothiocyanate derivatives mentioned above, in a concentration so that the daily consumption by a human adult is in the range of from 0.1mg/day to 50mg/day, preferably from 0.25mg/day to 30mg/day.

Typical examples of galenical formulations for use in accordance with the present invention are given below. The Examples are for the purpose of illustrating the invention and are not intended to limit the scope of the invention in any way.

The following Examples illustrate the invention further.

### Example 1

A tablet for the coadjuvant treatment of feminine acne is formulated to contain 5 mg of lycopene, 200 mg of vitamin E, 250 mg of vitamin C, 37.5 mg of resveratrol. The daily dose corresponds to said amounts in form of two tablets with half of said amounts each.

### Example 2

A tablet for the prevention of polycystic ovary syndrome is formulated to contain 2.5 mg of lycopene, 250 mg of vitamin E, 100 mg of vitamin C, 100mg silymarin.

## Claims

1. The use of lycopene in the manufacture of a composition for the primary and secondary prevention, incidence risk reduction, coadjuvant treatment or treatment of non-cancerous symptoms and/or pathologies, which are associated with, favored by or caused by androgen signalling, or which are sensitive to a reduction of androgen signalling, wherein the symptoms and/or pathologies are selected from the group consisting of: polycystic ovary syndrome, hyperandrogenic chronic anovulation, female infertility, ovarian hyperstimulation syndrome, amenorrhea, oligomenorrhea, obesity, accumulation of abdominal fat, hirsutism, menstrual disorder, hyperandiogenism, congenital adrenal hyperplasia (CAH) and stress induced imbalance of androgen signalling.

2. The use as in claim 1 of lycopene in combination with vitamin E.

3. The use as in claims 1 or 2 of lycopene in combination with vitamin E and/or vitamin C.

4. The use as in any one of claims 1 to 3 of lycopene in combination with one or more compounds selected from:
silymarin, silybin or equimolar amounts of derivatives, isosilybin or equimolar amounts of derivatives, silydianin or equimolar amounts of derivatives, silychristin or equimolar amounts of derivatives, saw palmetto or equimolar amounts of derivatives, free fatty acids (lauric acid, oleic acid, myristic acid, palmitic acid) or equimolar amounts of derivatives, phytosterols (sistosterol, campesterol, stigmasterol, cycloartenol, sitostanol, campestanol) or equimolar amounts of derivatives (long-chain fatty acid acyl ester, ferrulate ester, glycosides), genistein aglycone, genistein glucosides, genistein sulfates, genistein glucuronides, apigenin, quercetin or equimolar amounts of derivatives (quercetine glucosides, quercetin glucuronides, quercetine sulphates, methylquercetin (isohamnetin (3'-O-methylquercetin), tamarixetin(4'-O-methylquercetin)), myricetin, kampferol, resveratrol or equimolar amounts of derivatives, Curcumin (effects of *Curcuma Longa*) or equimolar amount of derivatives thereof (demethoxy-curcumin, bis-demethoxycurcumin, sodium curcumionate, bis-demethylcurcumin, tetrahydrocurcumin, hexahydrocurcumin, diacteylcurcumin, triethylcurcumin) and/or equimolar amount of ist main components components (curcumin (diferuloylmethane), demethoxycurcumin, bisdemethoxycurcumin) and/or derivatives thereof (glucuronides, sulfates), flufenamic acid, geldanamycin, extract of *Stephania hernandifolia* and/or or one or more of its components (e.g. 4-demethylhasubanonine, epistephanine), extract of *Myrica rubra* and/or one or more derivatives thereof and/or one or more of its components beeing diarylheptanoids (Quercetin, myricanone, myricanol, and myricetin) named acerogenin and their glycosides named aceroside and/or derivatives thereof, astaxanthin, β-carotene, β-cryptoxanthin, (-)-epigallocatechin gallate (EGCG) or (-)-epicatechin gallate (ECG) or equimolar amounts of derivatives, lutein, rhizoxin, palmitoyl rhizoxin, all-*trans* retinol, retinoic acids (*all-trans* retinoic acid and/or *13-cis* retinoic acid and/or *9-cis* retinoic acid) and/or one or more derivatives thereof (4-hydroxyphenylretinarmide or retinyl esters such as all-*trans* retinyl acetate); all-*trans* retinyl acetate, all-*trans* retinol palmitate, vitamin D2 (ergocalciferol), vitamin D3, (cholecalciferol), 1α, 25-dihydroxyvitamin D3, 25-hydroxyvitamin D3, 1α, 24R, 25-trihydroxyvitamin D3, 24, 25-dihydroxyvitamin D3, zeaxanthin, carnosic acid, carnosol, depudecin, eponemycin, dihydroeponemycin, epoxomicin, ergosterol, fisetin, fumagillin, lactacystin, luteolin, motuporamine C, ovalicin, radicicol, squalamine, isoliquiritin, isoliquiritigenin, very-long-chain omega-3 fatty acids (eicosapentaenoic acid [C20: 5, omega-3], decosahexaenoic acid [C22; 6, omega-3], polyunsaturated ω-3 fatty acids), shark cartilage extract, glucosinolate derivatives (Methylsulfinylalkyl glucosinolates (1-methylsulfinylmethyl glucosinolate, 2-methylsulfinylethyl glucosinolate, 3-methylsulfinylpropyl glucosinolate (glucoiberin), 4-methylsulfinylbutyl glucosinolate (glucoraphanin), 5-methylsulfinylpentyl glucosinolate (glucoalysin), 6-methylsulfinylhexyl glucosinolate, 7-methylsulfinylheptyl glucosinolate, 8-methylsulfinyloctyl glucosinolate, 9-methylsulfinylnonyl glucosinolate, 10-methylsulfinyldodecyl glucosinolate) or allyl glucosinolate (sinigrin) or indol-3-ylmethyl glucosinolate (glucobrassicin) or derivatives thereof (N-methoxyindol-3-ylmethyl glucosinolate (neoglucobrassicin), 4-hydroxyindol-3-ylmethyl glucosinolate (4-OH glucobrassicin), 4-methoxyindol-3-ylmethyl glucosinolate (4-CH₃O glucobrassicin)) or phenylethyl glucosinolate (gluconasturtiin) or 3-butenyl glucosinolate (gluconapin)), isothiocyanate derivatives (Methylsulfinylalkyl isothiocyanate (1-methylsulfinylmethyl isothiocyanate, 2-methylsulfinylethyl isothiocyanate, 3-methylsulfinylpropyl isothiocyanate, 4-methylsulfinylbutyl isothiocyanate (sulforaphane), 5-methylsulfinylpentyl isothiocyanate, 6-methylsulfinylhexyl isothiocyanate (6-HITC), 7-methylsulfinylheptyl isothiocyanate, 8-methylsulfinyloctyl isothiocyanate, 9-methylsulfinylnonyl isothiocyanate, 10-methylsulfinyidodecyl isothiocyanate) or allyl isothiocyanate, indole-3-ylmethylisothiocyanate, N-methoxy indole-3-ylmethylisothiocyanate, 4-hydroxy indole-3-ylmethylisothiocyanate, 4-methoxy indole-3-ylmethylisothiocyanate, 3-Indolemethanol, phenylethyl isothiocyanate (PEITC), 3-butenyl isothiocyanate).

5. The use as in claim 1, in which said pathology is hirsutism.

## Patentansprüche

1. Verwendung von Lycopin bei der Herstellung einer Zusammensetzung zur primären und sekundären Vorbeugung, Verringerung des Inzidenzrisikos, koadjuvanten Behandlung oder Behandlung von nichtkanzerösen Symptomen und/oder Pathologien, die mit Androgensignalwirkung verbunden, davon begünstigt oder davon verursacht sind, oder die für eine Verringerung der Androgensignalwirkung empfindlich sind, wobei die Symptome und/oder Pathologien ausgewählt sind aus der Gruppe, bestehend aus dem polyzystischen ovariellen Syndrom, hyperandrogener chronischer Anovulation, weiblicher Infertilität, dem ovariellen Hyperstimulationssyndrom, Amenorrhoe, Oligomenorrhoe, Obesitas, Akkumulation von abdominellem Fett, Hirsutismus, Menstruationsstörung, Hyperandrogenismus, kongenitaler Nebennierenhyperplasie (CAH) und stressinduziertem Ungleichgewicht der Androgensignalwirkung.

2. Verwendung gemäß Anspruch 1 von Lycopin in Kombination mit Vitamin E.

3. Verwendung gemäß Anspruch 1 oder 2 von Lycopin in Kombination mit Vitamin E und/oder Vitamin C.

4. Verwendung gemäß einem der Ansprüche 1 bis 3 von Lycopin in Kombination mit einer oder mehreren Verbindungen, ausgewählt aus:
Silymarin, Silybin oder äquimolaren Mengen von Derivaten, Isosilybin oder äquimolaren Mengen von Derivaten, Silydianin oder äquimolaren Mengen von Derivaten, Silychristin oder äquimolaren Mengen von Derivaten, Sägepalme oder äquimolaren Mengen von Derivaten, freie Fettsäuren (Laurinsäure, Oleinsäure, Myristinsäure, Palmitinsäure) oder äquimolaren Mengen von Derivaten, Phytosterole (Sistosterol, Campesterol, Stigmasterol, Cycloartenol, Sitostanol, Campestanol) oder äquimolaren Mengen von Derivaten (langkettige Fettsäureacylester, Ferrulatester, Glycoside), Genisteinaglycon, Genisteinglucoside, Genisteinsulfate, Genisteinglucuronide, Apigenin, Quercetin oder äquimolaren Mengen von Derivaten (Quercetinglucoside, Quercetinglucuronide, Quercetinsulfate, Methylquercetin (Isohamnetin (3'-O-Methylquercetin), Tamarixetin (4'-O-Methylquercetin)), Myricetin, Kampferol, Resveratrol oder äquimolaren Mengen von Derivaten, Curcumin (Wirkstoffe von *Curcuma Longa*) oder äquimolaren Mengen von Derivaten (Desmethoxycurcumin, Bisdesmethoxycurcumin, Natriumcurcumionat, Bisdesmethylcurcumin, Tetrahydrocurcumin, Hexahydrocurcumin, Diacteylcurcumin, Triethylcurcumin) und/oder einer äquimolaren Menge seiner Hauptkomponenten (Curcumin(diferuloylmethan), Desmethoxycurcumin, Bisdesmethoxycurcumin) und/oder Derivaten davon (Glucuronide, Sulfate), Flufenaminsäure, Geldanamycin, Extrakt von *Stephania hernandifolia* und/oder einer oder mehrerer seiner Komponenten (beispielsweise 4-Desmethylhasubanonin, Epistephanin), Extrakt von *Myrica rubra* und/oder einem oder mehreren Derivaten davon und/oder einer oder mehreren seiner Komponenten, die Diarylheptanoide sind (Quercetin, Myricanon, Myricanol und Myricetin), genannt Acerogenin, und ihren Glycosiden, genannt Acerosid, und/oder Derivaten davon, Astaxanthin, β-Carotin, β-Cryptoxanthin, (-)-Epigallocatechingallat (EGCG) oder (-)-Epicatechingallat (ECG) oder äquimolaren Mengen von Derivaten, Lutein, Rhizoxin, Palmitoylrhizoxin, all-*trans*-Retinol, Retinolsäuren (all-*trans*-Retinolsäure und/oder 13-*cis*-Retinolsäure und/oder 9-*cis*-Retinolsäure) und/oder einem oder mehreren Derivaten davon (4-Hydroxyphenylretinamid oder Retinylester, wie z. B. all-*trans*-Retinylacetat); all-*trans-*Retinylacetat, all-*trans*-Retinolpalmitat, Vitamin D2 (Ergocalciferol), Vitamin D3, (Cholecalciferol), 1α,25-Dihydroxyvitamin D3, 25-Hydroxyvitamin D3, 1α,24R,25-Trihydroxyvitamin D3, 24,25-Dihydroxyvitamin D3, Zeaxanthin, Carnosinsäure, Carnosol, Depudecin, Eponemycin, Dihydroeponemycin, Epoxomicin, Ergosterol, Fisetin, Fumagillin, Lactacystin, Luteolin, Motuporamin C, Ovalicin, Radicicol, Squalamin, Isoliquiritin, Isoliquiritigenin, sehr langkettigen omega-3-Fettsäuren (Eicosapentaensäure [C20:5,omega-3], Decosahexaensäure [C22:6,omega-3], mehrfach ungesättigte ω-3-Fettsäuren), Haifischknorpelextrakt, Glucosinolatderivativen (Methylsulfinylalkylglucosinolate (1-Methylsulfinylmethylglucosinolat, 2-Methylsulfinylethylqlucosinolat, 3-Methylsulfinylpropylglucosinolat (Glucoiberin), 4-Methylsulfinylbutylglucosinolat (Glucoraphanin), 5-Methylsulfinylpentylglucosinolat (Glucoalysin), 6-Methylsulfinylhexylglucosinolat, 7-Methylsulfinylheptylglucosinolat, 8-Methylsulfinyloctylglucosinolat, 9-Methylsulfinylnonylglucosinolat, 10-Methylsulfinyldodecylglucosinolat) oder Allylglucosinolat (Sinigrin) oder Indol-3-ylmethylglucosinolat (Glucobrassicin) oder Derivaten davon (N-Methoxyindol-3-ylmethylglucosinolat (Neoglucobrassicin), 4-Hydroxyindol-3-ylmethylglucosinolat (4-OH-Glucobrassicin), 4-Methoxyindol-3-ylmethylglucosinolat (4-CH₃O-Glucobrassicin)) oder Phenylethylglucosinolat (Gluconasturtiin) oder 3-Butenylglucosinolat (Gluconapin)), Isothiocyanatderivaten (Methylsulfinylalkylisothiocyanat (1-Methylsulfinylmethylisothiocyanat, 2-Methylsulfinylethylisothiocyanat, 3-Methylsulfinylpropylisothiocyanat, 4-Methylsulfinylbutylisothiocyanat (Sulforaphan), 5-Methylsulfinylpentylisothiocyanat, 6-Methylsulfinylhexylisothiocyanat (6-HITC), 7-Methylsulfinylheptylisothiocyanat, 8-Methylsulfinyloctylisothiocyanat, 9-Methylsulfinylnonylisothiocyanat, 10-Methylsulfinyldodecylisothiocyanat) oder Allylisothiocyanat, Indol-3-ylmethylisothiocyanat, N-Methoxyindol-3-ylmethylisothiocyanat, 4-Hydroxyindol-3-ylmethylisothiocyanat, 4-Methoxyindol-3-ylmethylisothiocyanat, 3-Indolmethanol, Phenylethylisothiocyanat (PEITC), 3-Butenylisothiocyanat).

5. Verwendung gemäß Anspruch 1, wobei die Pathologie Hirsutismus ist.

## Revendications

1. Utilisation de lycopène dans la fabrication d'une composition pour la prévention primaire et secondaire, la réduction du risque d'incidence, le traitement coadjuvant ou le traitement de symptômes et/ou de pathologies non cancéreux, qui sont associés à, favorisés par ou causés par la signalisation androgénique, ou qui sont sensibles à une réduction de signalisation androgénique, où les symptômes et/ou les pathologies sont choisis dans le groupe constitué de : syndrome des ovaires polykystiques, anovulation chronique hyperandrogénique, infertilité féminine, syndrome d'hyperstimulation ovarienne, aménorrhée, oligoménorrhée, obésité, accumulation de graisse abdominale, hirsutisme, trouble menstruel, hyperandrogénisme, hyperplasie surrénale congénitale (HSC) et déséquilibre de la signalisation androgénique induit par le stress.

2. Utilisation selon la revendication 1 du lycopène en combinaison avec de la vitamine E.

3. Utilisation selon la revendication 1 ou 2 du lycopène en combinaison avec de la vitamine E et/ou de la vitamine C.

4. Utilisation selon l'une quelconque des revendications 1 à 3 du lycopène en combinaison avec un ou plusieurs composés choisis parmi ;
la silymarine, la silybine ou des quantités équimolaires de dérivés, l'isosilybine ou des quantités équimolaires de dérivés, la silydianine ou des quantités équimolaires de dérivés, la silychristine ou des quantités équimolaires de dérivés, le saw palmetto ou des quantités équimolaires de dérivés, les acides gras libres (l'acide laurique, l'acide oléique, l'acide myristique, l'acide palmitique) ou des quantités équimolaires de dérivés, les phytostérols (le sistostérol, le campestérol, le stigmastérol, le cycloarténol, le sitostanol, le campestanol) ou des quantités équimolaires de dérivés (ester d'acyle d'acide gras à longue chaîne, ester ferrulate, glycosides), la génistéine aglycone, les glucosides de génistéine, les sulfates de génistéine, les glucuronides de génistéine, l'apigénine, la quercétine ou des quantités équimolaires de dérivés (glucosides de quercétine, glucuronides de quercétine, sulfates de quercétine, méthylquercétine (isohamnétine (3'-O-méthylquercétine), tamarixétine (4'-O-méthylquercétine)), la myricétine, le kampférol, le resvératrol ou des quantités équimolaires de dérivés, la curcumine (effets de *Curcuma Longa*) ou des quantités équimolaires de dérivés de celle-ci (déméthoxycurcumine, bis-déméthoxycurcumine, curcumionate de sodium, bis-déméthylcurcumine, tétrahydrocurcumine, hexahydrocurcumine, diacétylcurcumine, triéthylcurcumine) et/ou des quantités équimolaires de ses composants principaux (curcumine (diféruloylméthane), déméthoxycurcumine, bis-déméthoxycurcumine) et/ou des dérivés de ceux-ci (glucuronides, sulfates), l'acide flufénamique, la geldanamycine, l'extrait de *Stephania hernandifolia* et/ou un ou plusieurs de ses composants (par exemple 4-déméthylhasubanonine, épistéphanine), l'extrait de *Myrica rubra* et/ou un ou plusieurs dérivés de celui-ci et/ou un ou plusieurs de ses composants étant les diarylheptanoïdes (quercétine, myricanone, myricanol et myricétine) nommés l'acérogénine et leurs glycosides nommés l'acéroside et/ou les dérivés de ceux-ci, l'astaxanthine, le β-carotène, la β-cryptoxanthine, le gallate de (-)-épigallocatéchine (EGCG) ou le gallate de (-)-épicatéchine (ECG) ou des quantités équimolaires de dérivés, la lutéine, la rhizoxine, la palmitoylrhizoxine, le tout-*trans*-rétinol, les acides rétinoïques (acides *tout*-*trans*-rétinoïque et/ou acide *13-cis*-rétinoïque et/ou acide *9-cis-*rétinoïque) et/ou un ou plusieurs dérivés de ceux-ci (4-hydroxyphénylrétinamide ou esters de rétinyle tels que l'acétate de *tout-trans-*rétinyle) ; l'acétate de *tout-trans*-rétinyle, le palmitate de *tout-trans*-rétinol, la vitamine D2 (ergocalciférol), la vitamine D3 (cholécalciférol), la 1α,25-dihydroxyvitamine D3, la 25-hydroxyvitamine D3, la 1α,24R,25-trihydroxyvitamine D3, la 24,25-dihydroxyvitamine D3, la zéaxanthine, l'acide carnosique, le carnosol, la dépudécine, l'éponémycine, la dihydroéponémycine, l'époxomicine, l'ergostérol, la fisétine, la fumagilline, la lactacystine, la lutéoline, la motuporamine C, l'ovalicine, le radicicol, la squalamine, l'isoliquiritine, l'isoliquiritigénine, les acides gras oméga-3 à très longue chaîne (acide eicosapentaénoïque [C20 : 5, oméga-3], l'acide décosahexaénoïque [C22 : 6, oméga-3], les acides gras ω-3 polyinsaturés), l'extrait de cartilage de requin, les dérivés de glucosinolate (les glucosinolates de méthylsulfinylalkyle (glucosinolate de 1-méthylsulfinylméthyle, glucosinolate de 2-méthylsulfinyléthyle, glucosinolate de 3-méthylsulfinylpropyle (glucoibérine), glucosinolate de 4-méthylsulfinylbutyle (glucoraphanine), glucosinolate de 5-méthylsulfinylpentyle (glucoalysine), glucosinolate de 6-méthylsulfinylhexyle, glucosinolate de 7-méthylsulfinylheptyle, glucosinolate de 8-méthylsulfinyloctyle, glucosinolate de 9-méthylsulfinylnonyle, glucosinolate de 10-méthylsulfinyldodécyle) ou le glucosinolate d'allyle (sinigrine) ou le glucosinolate d'indol-3-ylméthyle (glucobrassicine) ou les dérivés de ceux-ci (glucosinolate de N-méthoxyindol-3-ylméthyle (néoglucobrassicine), glucosinolate de 4-hydroxyindol-3-ylméthyle (4-OH glucobrassicine), glucosinolate de 4-méthoxyindol-3-ylméthyle (4-CH₃O glucobrassicine)) ou le glucosinolate de phényléthyle (gluconasturtiine) ou le glucosinolate de 3-butényle (gluconapine)), les dérivés d'isothiocyanate (l'isothiocyanate de méthylsulfinylalkyle (isothiocyanate de 1-méthylsulfinylméthyle, isothiocyanate de 2-méthylsulfinyléthyle, isothiocyanate de 3-méthylsulfinylpropyle, isothiocyanate de 4-méthylsulfinylbutyle (sulforaphane), isothiocyanate de 5-méthylsulfinylpentyle, isothiocyanate de 6-méthylsulfinylhexyle (6-HITC), isothiocyanate de 7-méthylsulfinylheptyle, isothiocyanate de 8-méthylsulfinyloctyle, isothiocyanate de 9-méthylsulfinylnonyle, isothiocyanate de 10-méthylsulfinyldodécyle) ou l'isothiocyanate d'allyle, l'isothiocyanate d'indol-3-ylméthyle, l'isothiocyanate de N-méthoxyindol-3-ylméthyle, l'isothiocyanate de 4-hydroxyindol-3-ylméthyle, l'isothiocyanate de 4-méthoxyindol-3-ylméthyle, le 3-indoleméthanol, l'isothiocyanate de phényléthyle (PEITC), l'isothiocyanate de 3-butényle).

5. Utilisation selon la revendication 1, **caractérisée en ce que** ladite pathologie est l'hirsutisme.
